# EUROPEAN PATENT APPLICATION

(11) **EP 4 461 207 A1**
(43) Date of publication of application: **13.11.2024**
(21) Application number: 24175123.9
(22) Date of filing: 10.05.2024
(51) Int. Cl.: A61B 3/00, A61B 3/12

(54) **SYSTEMS AND METHODS FOR BINOCULAR EYE FUNDUS VIEWING**

(30) Priority: 10.05.2023 US 202318315434
(71) Applicant: Pacific University, Forest Grove, OR 97716 (US)
(72) Inventor: FISCHER, Mark, Dubuque, 52003 (US); CHRISTENSEN, Griffin, River Falls, 54022 (US); SORIA, Juan Andres, Hillsboro, 97123 (US); GAY, Lila K., Forest Grove, 97116 (US); FISCHER O'DAY, Terry, Dodgeville, 53533 (US); KEPPINGER, Mark Norman, Hillsboro, 97123 (US)
(74) Representative: Maikowski & Ninnemann Patentanwälte Partnerschaft mbB

(57) **Abstract**

The invention relates to a system for binocular viewing of an eye fundus, comprising:
a casing comprising a first section coupled to a second section;
a light source coupled to a patient-facing end of the first section and configured to provide light along a light path towards the eye fundus; and
a lens receiving opening formed into a practitioner-facing end of the second section of the casing, the lens receiving opening configured to receive and hold a lens within the second section.

The invention further relates to a method for binocular viewing of an eye fundus, comprising: providing light directly from a light source to the eye fundus;
receiving the light from the eye fundus at a lens; and
generating a binocular image of the eye fundus via the light received at the lens.

## Description

### Field

The present description relates generally to systems and methods for binocular viewing of an eye fundus. In one or more examples, the disclosure relates to systems and methods for binocular indirect ophthalmoscopy (BIO).

### Background/Summary

Binocular viewing of an eye fundus is used to monitor the eye health of a patient. The fundus of the eye is a posterior region of the eye opposite the pupil that comprises several anatomical features, such as the retina, choroid, optic nerve and macula. It is common practice to perform binocular examination of the eye fundus regularly to monitor for lesions and degenerative disease. For example, binocular viewing of the eye fundus provides depth information that may assist in detecting possible retinal issues, such as bleeds, retinal detachment, or ischemic events, for example. Depth perception is particularly needed in order to accurately determine swelling or endemic regions of the eye fundus, for example.

Traditional approaches for binocular viewing of the eye fundus include binocular indirect ophthalmoscopy (BIO). Standard BIO systems comprise head-mounted equipment worn by a viewer and a handheld lens. During traditional BIO examinations, a practitioner holds a condenser lens in front of a patient's eye while the practitioner wears a head-mounted light source. The head-mounted light source worn by the practitioner is used to direct light through the hand-held lens, and into the patient's eye. Light is then reflected off the fundus of the patient's eye and passed back through the hand-held lens for a second time to generate a binocular image of the eye fundus, while additional head-mounted mirrors and lenses allow the practitioner to view the binocular image. In this way, a dual pass approach has previously been used for generating a binocular image of the eye fundus, as the light is passed through the same lens two times.

For example, looking to Schepens et al. in U.S. Patent No. 5,400,092, therein a dual pass approach for generating a binocular view of the eye fundus is disclosed, where light from the head-mounted equipment worn by a clinician is passed through a lens a first time. The light that has passed through the lens is then reflected off the eye fundus of the patient, and passed back through the lens a second time to generate the binocular view of the eye fundus.

However, the inventors herein have recognized several potential issues with such previous systems. As one example, these traditional BIO systems are large and require components that may be difficult to manipulate and transport. Further, due to the number of components, these traditional BIO systems are costly and may lack adjustability desired by the user. Moreover, dual pass light approaches may be prone to unintended distortion due to the extended light path.

Further still, there is increased interest in recording images of the eye fundus. For example, such images may help to track disease incidence and progression in a patient's chart, as well as improve accessibility of care for regions with fewer resources. Traditional BIO systems are not suitable for direct image capture via another image capture device because such direct image capture devices interrupt the dual pass light path. There are fundus cameras available. However, such fundus cameras do not have binocular capabilities. There are also adapters for traditional BIO systems to capture images. However, these adapters are an added cost to the already high cost of the BIO system.

In one example, the issues described above may be addressed by a system for binocular viewing of an eye fundus comprising a casing that includes a light source section and a lens receiving section. In this way, binocular viewing of the eye fundus may be achieved without the need for a head-mounted light source and by only a single pass of the light through the lens.

As one example, the light source may be a light ring that surrounds a light receiving aperture, and the lens receiving section may be configured to couple a lens within the lens receiving section. The light ring may further be adjustable, such that one or more of a focus and a color of light emitted from the light ring may be set by a user. Via such features, the system is configured to generate a binocular view of the eye fundus with a single pass of light through the lens receiving section of the casing. In particular, light may be emitted from the light ring, reflected off the eye fundus of the patient, passed through the light receiving aperture, and then directed for a single pass through the lens receiving section to generate the binocular view. It is noted that the binocular view may also be referred to herein as a binocular image.

The system disclosed may thus be more compact than previous BIO systems and be manufactured a reduced cost compared to previous BIO systems, at least in part due to the elimination of a head-mounted light source. Furthermore, the system disclosed provides an adjustability that may help generate higher quality binocular views of the eye fundus than may otherwise be possible. Additionally, the configuration of the disclosed systems and methods allow for an image capture device (e.g., a mobile device) to directly capture the binocular image generated.

It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### Brief Description of the Drawings

FIG. 1 shows a schematic for dual pass binocular viewing of an eye fundus according to the prior art.
FIG. 2 shows a schematic for single pass binocular viewing of an eye fundus according to the present disclosure.
FIG. 3 shows a perspective view of a binocular eye fundus viewing system, according to one or more examples of the present disclosure.
FIG. 4 shows a cut-away view of the binocular eye fundus viewing system, according to one or more examples of the present disclosure.
FIG. 5 shows an exploded view of the binocular eye fundus viewing system, according to one or more examples of the present disclosure.
FIG. 6 shows an exploded view of the lens receiving section casing from the binocular eye fundus viewing system, according to one or more examples of the present disclosure.
FIG. 7 shows a view of an electronic board unit from the binocular eye fundus viewing system, according to one or more examples of the present disclosure.
FIG. 8 shows a partial cutaway view of the light source configuration from the binocular eye fundus viewing system, according to one or more examples of the present disclosure.
FIG. 9 shows a perspective view of a fiber optics support structure from the binocular eye fundus viewing system, according to one or more examples of the present disclosure.
FIG. 10 shows a partial cutaway view of the fiber optics support structure from the binocular eye fundus viewing system, according to one or more examples of the present disclosure.
FIG. 11 shows a view of a deflection ring of the deflection ring device for the binocular eye fundus viewing system, according to one or more examples of the present disclosure.
FIG. 12 shows an exploded view of the deflection ring device for the binocular eye fundus viewing system, according to one or more examples of the present disclosure.
FIG. 13A shows a first partial view of an angle adjustment carried out via the deflection ring device for the binocular eye fundus viewing system, according to one or more examples of the present disclosure.
FIG. 13B shows a second view of the angle adjustment carried out via the deflection ring device for the binocular eye fundus viewing system in a second position, according to one or more examples of the present disclosure.
FIG. 14 shows a first view of the light source section of the casing from the binocular eye fundus viewing system, according to one or more examples of the present disclosure.
FIG. 15 shows a second view of the light source section of the casing from the binocular eye fundus viewing system, according to one or more examples of the present disclosure.
FIG. 16 shows a first circuit diagram of the light source configuration, according to one or more examples of the present disclosure.
FIG. 17 shows a second circuit diagram of the light source configuration, according to one or more examples of the present disclosure.
FIG. 18 shows a flow chart of an example method, according to one or more examples of the present disclosure.

### Detailed Description

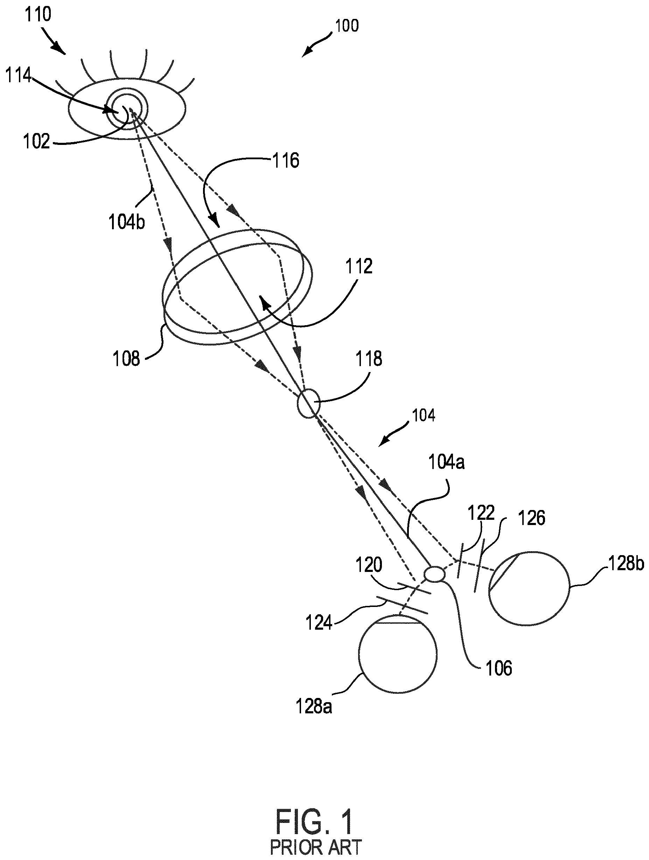

The following description relates to systems and methods for binocular eye fundus viewing, for example BIO. As shown at FIG. 1, prior art approaches for binocular eye fundus viewing include a dual pass approach in which light is passed through a lens two times. Further, as seen in FIG. 1, prior art systems for binocular eye fundus viewing (e.g., prior art BIO systems) utilize head-mounted equipment and handheld equipment.

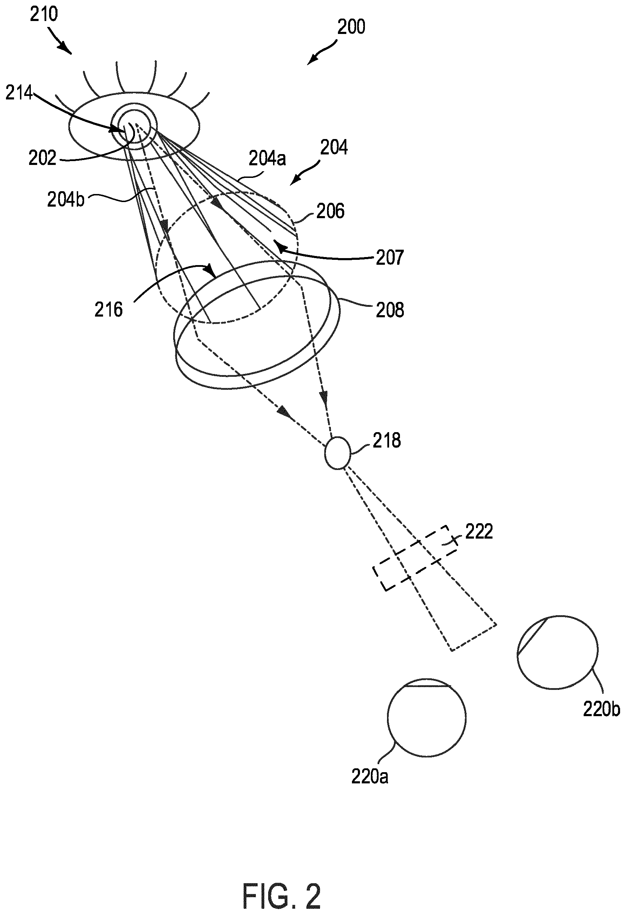

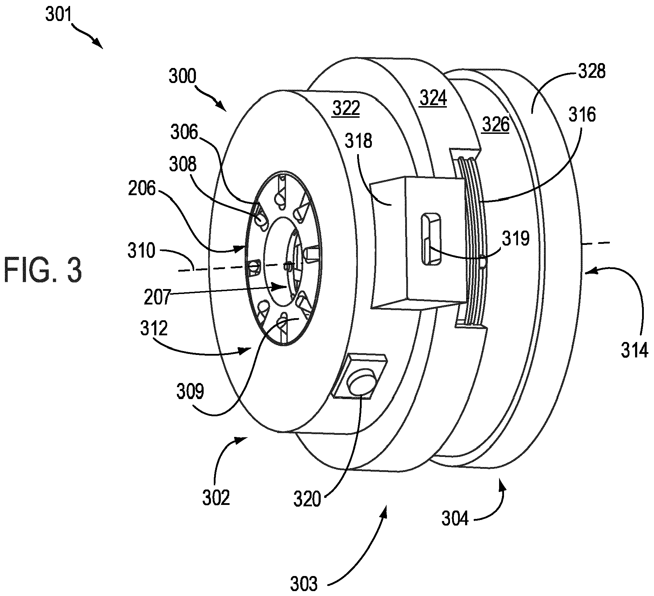

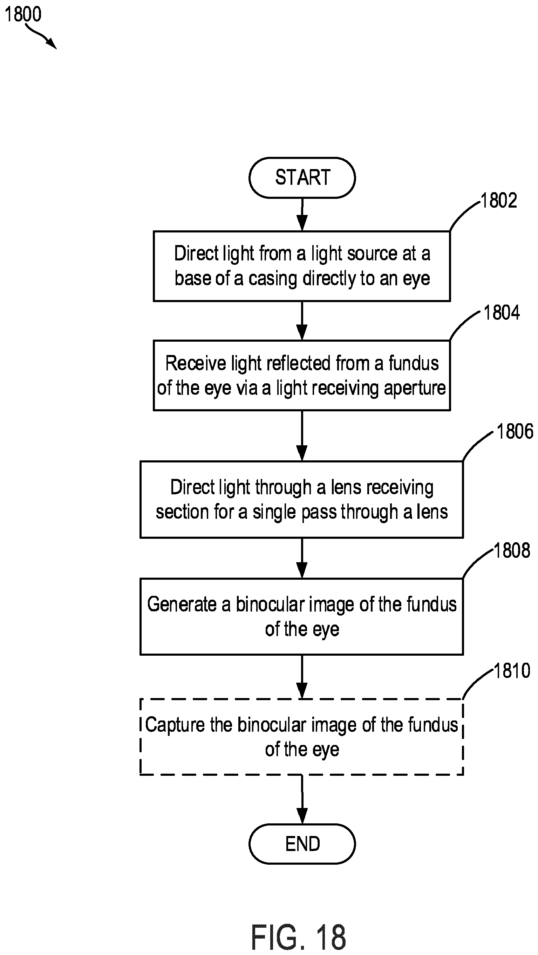

In contrast, as seen at FIG. 2 and FIG. 18, the systems and methods disclosed herein for binocular eye fundus viewing (including BIO) instead use a single pass approach in which light is only passed through a lens one time. As further illustrated at FIG. 2, the systems and methods disclosed herein do not require a head-mounted equipment such as the head-mounted light source required in the prior art example at FIG. 1. Rather, as seen at FIG. 3, the systems disclosed herein comprise a casing with a light source section and a lens receiving section.

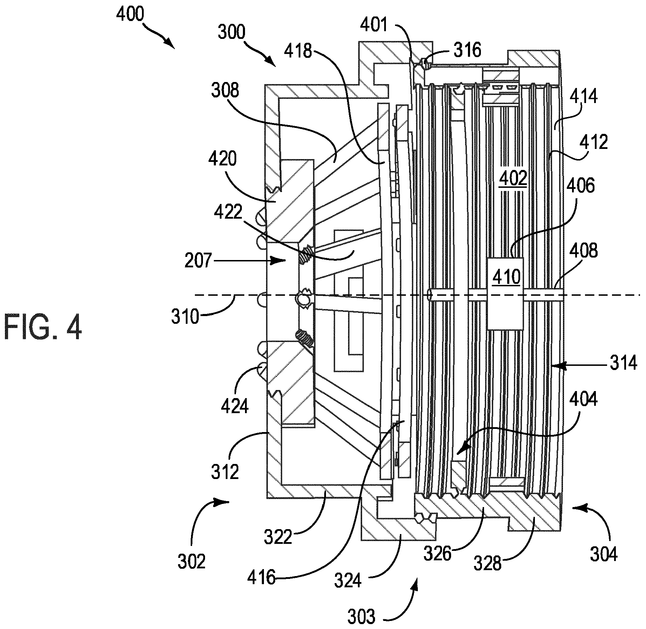

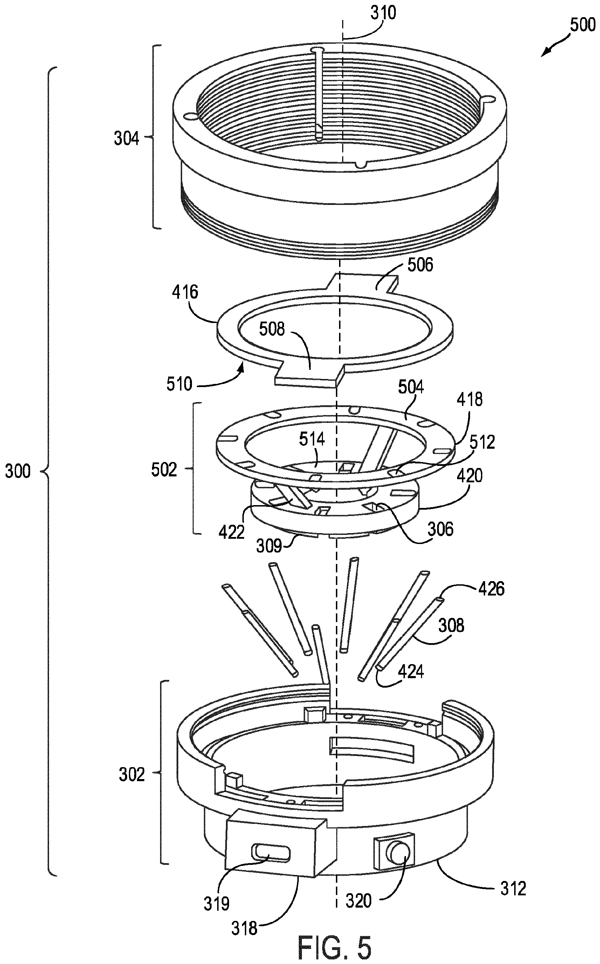

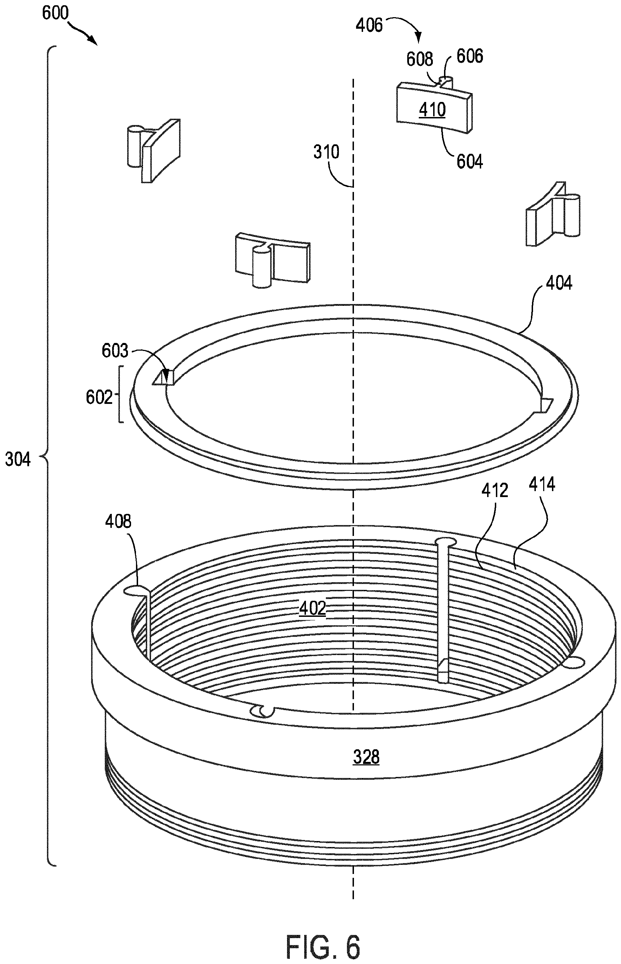

The lens receiving section may comprise one or more adjustable locking mechanisms configured to couple a lens within the lens receiving section, as seen at FIG. 6. The light source section may comprise a light ring that surrounds a light receiving aperture. As seen at FIG. 4, for example, the light receiving aperture may open into an interior of the light source section, where the interior of the light source section is configured to allow light to pass through to the lens receiving section. The system may further comprise components that are configured to guide fiber optic cables of the light ring in a manner that allows light to be received at the lens receiving section via the light source section without obstruction, as illustrated at FIGS. 5 to 15 of the system, for example.

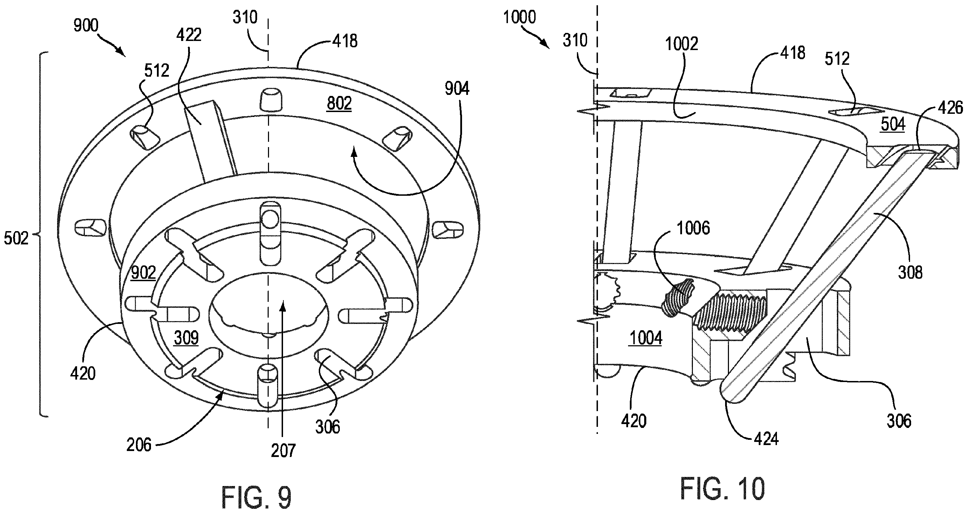

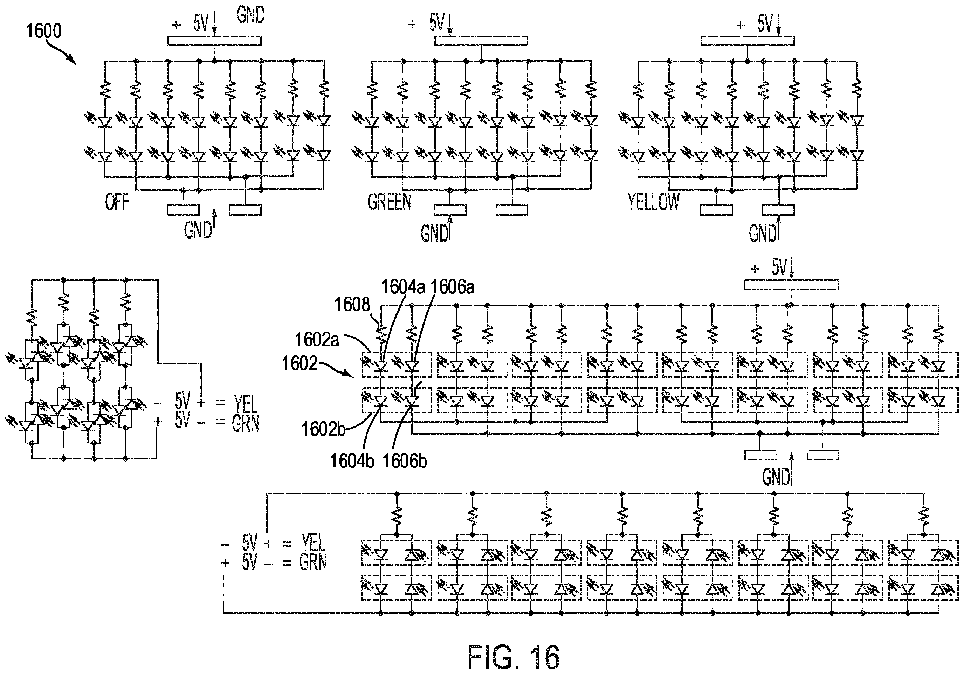

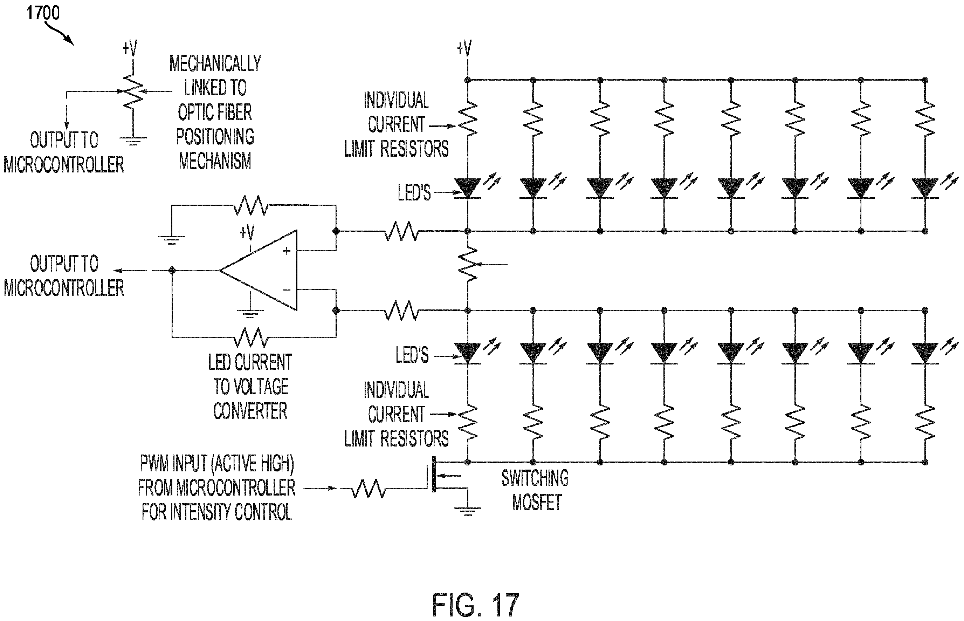

Further, as shown at FIGS. 10-13B, an angle for each of the fiber optic cables of the light ring may be adjustable to focus light emitted from the light ring. Further adjustability as to the color and brightness of the light emitted from the light ring may be provided, as shown at the circuit diagrams shown at FIG. 16 and FIG. 17.

FIGS. 3-15 are shown approximately to scale. Further, for purposes of discussion, the figures are described collectively. Thus, similar features across the figures may be assigned similar reference numerals and may not be re-introduced. Furthermore, discussion directed towards one figure may also include explanation as to the relationship of the one figure with features found in other figures.

Turning now to FIG. 1, FIG. 1 shows a schematic 100 for binocular viewing an eye fundus 102, according to the prior art. In particular, the schematic 100 illustrates an approach for binocular indirect ophthalmoscopy (BIO) according to the prior art.

As may be seen in FIG. 1, binocular viewing of the eye fundus 102 according to the prior art relies upon a dual pass approach. In the dual pass approach of the prior art, light 104 generated from a light source 106 is directed through a lens 108 two times. It is noted that the light 104 refers to both 104a and 104b, where the light 104a represented by solid lines is light that has not been reflected off of the eye fundus 102. The light 104b represented by dash lines is light that has been reflected off of the eye fundus 102.

The first time the light 104a is directed through the lens 108, also referred to herein as a first pass, the light 104a is directed from the light source 106 through the lens 108 upstream of an eye 110 of the patient. That is, the first pass involves light being passed through the lens 108 without having passed through the eye 110 of the patient.

The second time the light 104b is directed through the lens 108, also referred to herein as a second pass, follows reflection of the light off of the eye fundus 102 of the patient. In particular, after the first pass, the light 104a that has already passed through the lens 108 once is directed to the eye 110 of the patient, reflected off the eye fundus 102 of the patient, and then the light 104b reflected off the eye fundus 102 directed back through the lens 108 for the second pass.

As seen in the dual pass approach at FIG. 1, for the first pass, the light source 106 generates light 104a, and the light 104a is directed from the light source 106 through the lens 108. It is noted that the lens 108 may be a condensing lens, for example.

For the first pass, light 104a may be directed from the light source 106 through approximately a center of the lens 108. Further, the light 104a for the first pass is incident on a first side 112 of the lens 108, where the first side 112 of the lens 108 faces away from the eye 110 of the patient and towards the viewer.

After the light 104a completes the first pass through the lens 108, the light 104a then reaches the eye 110 of the patient and may pass through a pupil 114 of the eye 110. For example, the lens 108 may condense light 104a received from the light source 106 towards the pupil 114 of the eye 110. The light 104a passed through the pupil 114 of the eye 110 may then reflect off the eye fundus 102, and the light 104b reflected off the eye fundus 102 may be directed back towards the lens 108 for the second pass.

For the second pass, the light 104b reflected off the eye fundus 102 of the patient is passed through the lens 108 for the second time. The light 104b reflected off the eye fundus 102 of the patient may be passed through the lens 108 near a perimeter of the lens 108 in the second pass, for example. It is noted that the light is incident on a second side 116 of the lens 108 for the second pass, where the second side 116 of the lens 108 faces towards the eye 110 of the patient and away from the viewer.

The light 104b reflected off the eye fundus 102 and directed through the lens 108 for the second pass creates an aerial image 118 of the eye fundus 102, where the aerial image 118 is a true image that is horizontally and laterally inverted. It is noted that the aerial image 118 is positioned between the lens 108 and the viewer. The aerial image 118 is then reflected and inverted via a first mirror 120 and a second mirror 122, and observed through a first powered convex lens 124 and a second power convex lens 126 by eyes 128a, 128b of the viewer. In this way, the prior art approach of FIG. 1 provides a stereoscopic view of the eye fundus 102.

The dual pass approach shown in FIG. 1 is traditionally implemented via head-mounted equipment worn by the viewer in combination with a handheld lens that is held by the viewer.

The head-mounted equipment may comprise the light source 106, first mirror 120, second mirror 122, the first powered convex lens 124, and the second power convex lens 126. The light source 106 may be mounted on the viewer's head and positioned at a location above and directly between viewer's own eyes 128a, 128b to guide the light 104a through the handheld lens, where the handheld lens may comprise the lens 108, for example.

Via such traditional approaches, the hand-held lens (e.g., lens 108) is held by the clinician at a focal length of the hand-held lens from the eye 110 of the patient, and the clinician further adjusts the head-mounted equipment (including at least the light source 106) to carry out BIO examination of the eye fundus. Thus, for such approaches, the clinician is required to coordinate both head-mounted equipment in combination with a hand-held lens to obtain a stereoscopic view of the eye fundus 102.

Looking now to FIG. 2, FIG. 2 shows a schematic 200 for binocular viewing of an eye fundus 202 according to the present disclosure. In particular, the schematic 200 shown at FIG. 2 may be for BIO.

As may be seen in FIG. 2, the disclosure utilizes a single pass approach for binocular viewing of the eye fundus 202 of a patient. Thus, in contrast to the prior art approach disclosed at FIG. 1, which passes light 104a, 104b through the lens 108 two times to provide a view of the eye fundus 102, the present disclosure at FIG. 2 instead only passes light 204 through a lens 208 one time. It is noted that the light 204 refers to both 204a and 204b, where the light 204a represented by solid lines is light that has not been reflected off of the eye fundus 202. The light 204b represented by dash lines is light that has been reflected off of the eye fundus 202. In this way, the single light path and optical physics achieved via the disclosed system are illustrated via FIG. 2.

In the single pass approach disclosed at FIG. 2, a light source 206 first directs light 204a to the eye 210 of the patient. In particular, the light 204a from the light source 206 may be directed into a pupil 214 of the eye 210. In at least one example, the light source 206 may be a light ring and thus may also be referred to herein as a light ring 206. The light ring 206 may focus the light 204a towards the pupil 214 of the eye 210. To that end, the focus of the light ring 206 may be adjustable in one or more example, as described in more detail herein below.

As may be seen in FIG. 2, the light 204a from the light source 206 is not passed through any lens prior to being directed into the eye 210. Rather, the light 204a from the light source 206 may be provided from the light source 206 directly to the eye 210 of the patient without passing through any lens prior to reaching the eye 210 of the patient. Moreover, the light 204a generated by the light source 206 may be provided in a direction away from the lens 208, and the light source 206 may be positioned downstream of the lens 208. This is in contrast to prior art approaches such as those disclosed at FIG. 1. As seen in FIG. 1, the light source 106 is upstream of the lens 108 so that light 104a from the light source 106 can be directed towards the lens 108 and passed through the lens 108 before the light 104a reaches the eye 110 of the patient.

Looking back to FIG. 2, the light 204a directed towards the pupil 214 of the eye 210 in FIG. 2 passes through the pupil 214 and is reflected off the eye fundus 202. The light 204b reflected off of the eye fundus 202 then passes back through the pupil 214 and is directed towards the light source 206. The light 204b reflected off the eye fundus 202 may then pass through a light receiving aperture 207, where the light receiving aperture 207 may be an opening surrounded by the light source 206. For example, the light receiving aperture 207 may be an opening surrounded by the light source 206 in a light ring configuration, as described in more detail herein below.

After the light 204b reflected off the eye fundus 202 is passed through the light receiving aperture 207, the light 204b is then received at the lens 208 for a first pass through the lens 208, where the light 204b is incident on a side 216 of the lens 208 facing towards the eye 210 for the first pass. It is noted that the side 216 may be referred to as a light receiving side of the lens 208, in one or more examples. The first pass of the light 204b through the lens 208 then occurs. It is noted that the light 204b may be received near a perimeter of the lens 208 for the first pass, for example, and that the lens 208 may be a condenser lens.

The light 204b passed through the lens 208 generates a binocular image 218 of the eye fundus 202. For example, the binocular image 218 (also referred to herein as a binocular view) may be an aerial image of the eye fundus 202, where the aerial image has binocularity. The aerial image may be a true image that is horizontally and laterally inverted that is positioned between the lens and the viewer.

The binocular image 218 is then able to be viewed via a first eye 220a and a second eye 220b of the viewer. Additionally, in one or more examples, the binocular image 218 may be captured via an image capture device 222 (e.g., camera, phone, video, etc.). In such example where the binocular image 218 is captured, it is noted that both binocularity and image capturing capabilities are achieved at the same time using a single light pass.

Thus, as shown in FIG. 2, the first pass of the light 204b through the lens 208 is after the light 204b has already been reflected off of the eye fundus 202. Further, the first pass through the lens 208 is the only pass of the light 204b through the lens 208 used to generate the binocular image of the eye fundus 202.

In this way, a binocular image is generated via a single pass of the light 204 through the lens 208, as shown in FIG. 2, rather than the binocular image generated in FIG. 1 via two passes of the light 104 through the lens 108.

In particular, as illustrated in FIG. 2, light 204a is first directed towards the eye 210 of the patient and focused towards the pupil 214 of the eye 210 (for example, via a light ring configuration). The light 204a directed towards the eye 210 of the patient is not passed through the lens 208 before the light 204a reaches the eye 210. Then, the light 204a is reflected off the eye fundus, and the light 204b that is reflected off of the eye fundus is received at the light receiving aperture 207 (for example, an opening surrounded by the light ring configuration). After passing the light 204b through the light receiving aperture 207, the light 204b is subsequently directed through the lens 208 for the first pass, and only pass, to generate a binocular image 218 of the eye fundus 202.

Further, as is made clear looking to FIG. 3, the FIG. 2 approach for binocular viewing of the eye fundus 202 is able to be carried out via a system that does not require a head-mounted light source and is substantially compact compared to traditional BIO systems.

Looking to FIG. 3, FIG. 3 shows a perspective view 301 of a binocular eye fundus viewing system 300 (also referred to herein as the system 300) according to one or more examples of the present disclosure. The system 300 may be a BIO system. In one or more examples, the system 300 at FIG. 3 may be used to carry out the steps disclosed at FIG. 2 for the single pass generation of the binocular image of the eye fundus.

The binocular eye fundus viewing system 300 comprises a casing 303 that is generally cylindrical in shape, where the casing 303 comprises a first section and a second section, referred to as a light source section 302 and a lens receiving section 304, respectively. As may be seen in FIG. 3, the casing 303 of the system 300 may comprise sections that vary in diameter. For example, as shown in FIG. 3, the casing 303 may include four ring sections, including a first ring section 322, a second ring section 324, a third ring section 326, and a fourth ring section 328 that vary in diameter. It is noted that the system 300 is configured as a handheld system. Thus, the variation in diameter along the length of the casing 303 enables improved tactile support, so that a user may more easily hold and balance the system 300 while conducting an exam.

Looking to the light source section 302, the light source section 302 may include the first ring section 322 and the second ring section 324, where the light source 206 is at an end of the light source section 302.

The light source 206 may be in a light ring configuration and thus is also referred to herein as the light ring 206 of the system 300. In one or more examples, the light ring 206 may surround the light receiving aperture 207. In at least one example, the light ring 206 may comprise a plurality of light emission openings 306 through which light (e.g., the light 204a discussed at FIG. 2) is emitted via corresponding fiber optic cables 308.

In particular, each of the light emission openings 306 may have one of the fiber optic cables 308 positioned therein that emits light there through. The light emission openings 306 may be formed into a surface 309 of the light ring 206 that is part of a fiber optics support structure (described in further detail herein). The plurality of light emission openings 306 and the corresponding fiber optic cables 308 may circumferentially surround the light receiving aperture 207, in one or more examples. Further, as described in further detail herein below, the light ring 206 may be adjustable, so as to allow a focus of the light (e.g., light 204a) output by the light ring 206 to be adjusted as desired by a clinician. To that end, the light emission openings 306 may be configured to allow adjustment of a position of the fiber optic cables 308 within the light emission openings 306 for such focus adjustment, in one or more examples.

The light ring 206 may surround a central axis 310 of the system 300, where the central axis 310 extends in an axial direction through a center of the system 300. The central axis 310 may act as a centerline for the light ring 206. The light ring 206 may further form part of an exterior surface of the system 300 at an end face 312 of the binocular eye fundus viewing system 300. In one or more examples, the end face 312 may be circular in cross-section. Thus, the end face 312 and the light ring 206 may be concentric rings that surround and define the light receiving aperture 207. It is noted that the end of the system 300 comprising the end face 312 and the light source 206 may be referred to as a base or a patient-facing end of the system 300. The opposite end of the system 300 at which the lens receiving opening 314 is located may be referred to as a provider-facing end.

In addition to the light ring 206, the light source section 302 may further comprise a charging section 318, as well as a user input device 320. The charging section 318 may enable charging of a battery for the light ring 206 via a charging port 319. The charging port 319 may be a USB port, micro USB port, or other suitable port, in one or more examples. As to the user input device 320, the user input device 320 may comprise a mechanical device such as a button or a switch, or other user input option that allows a user to adjust operation of the light ring 206. For example, the user input device 320 may allow for the light ring 206 to be turned on and off. Additionally, the user input device 320 may further allow other lighting modes for the light ring 206 to be activated, such as one or more of different light color modes and different light intensity modes.

The light source section 302 may be coupled to the lens receiving section 304, where the light source section 302 may define a lens receiving opening 314. In one or more examples, the light source section 302 may be coupled to the lens receiving section 304 via an engagement section 316. The engagement section 316 may comprise an engagement surface to mechanically couple the light source section 302 and the lens receiving section 304 together. For example, the engagement section 316 may be threading in one or more examples, where the threading may form part of an exterior surface of the lens receiving section 304.

Looking to FIG. 4, for example, FIG. 4 shows a cut-away view 400 of the binocular eye fundus viewing system 300 of FIG. 3.

As can be seen in FIG. 4, the engagement section 316 is configured to detachably couple the light source section 302 and the lens receiving section 304 of the casing 303. For example, the engagement section 316 may comprise threading, and the threading may couple the light source section 302 to a corresponding engagement surface 401 of the lens receiving section 304. As shown in FIG. 4, the corresponding engagement surface 401 may be formed into an interior surface of the lens receiving section 304 at the second ring section 324 of the casing 303. The internal diameter of the second ring section 324 is thus greater than an outer diameter of the third ring section 326 of the casing to allow the engagement section 316 and the corresponding engagement surface 401 form a junction at which the light source section 302 and the lens receiving section 304 can be detachably coupled.

In addition to the engagement section 316, the lens receiving section 304 may further comprise lens coupling components within an interior of the lens receiving section 304, where the lens coupling components include one or more of an internal engagement section 402, locking ring 404, spacers 406, and spacer grooves 408 are configured to engage and hold a lens (e.g., lens 208) in place within the lens receiving section 304 of the casing 303. That is, the lens is held within the lens receiving section 304 of the casing such that the casing 303 surrounds the lens. In this way, the lens receiving section 304 forms a sleeve that may surround and secure the lens.

The lens coupling components may form adjustable locking mechanisms for holding the lens in place. In particular, the internal engagement section 402 and the locking ring 404 may form a first adjustable locking mechanism, and the spacers 406 and spacer grooves 408 may form a second adjustable locking mechanism for holding a lens in place. In this way, the system 300 may comprise two different types of adjustable locking mechanisms. Alternatively, in one or more examples only one of the adjustable locking mechanisms may be included in the system 300. Further, more than two adjustable locking mechanisms may be included in the system 300 to hold the lens in place without departing from the scope of the present disclosure.

Concerning the first adjustable locking mechanism comprising the internal engagement section 402 and the locking ring 404, the internal engagement section 402 may comprise threading that includes ridges 412 and grooves 414 formed into the interior walls of the lens receiving section 304 of the casing 303, in at least one example. In one or more examples, other forms of engagement may be possible for the internal engagement section 402. For example, one or more of screws, friction fittings, and magnetic coupling elements may be possible in addition to or as an alternative to the threading for the internal engagement section 402. The internal engagement section 402 may be configured to engage with the locking ring 404 enabling the system 300 to accommodate various lenses, as described in further detail herein below.

As to the second adjustable locking mechanism, the second adjustable locking mechanism may comprise one or more spacers 406 and spacer grooves 408. It is noted that the view at FIG. 4 shows only one of the spacers 406 and one of the spacer grooves 408. In particular, the view at FIG. 4, shows a lens engagement surface 410 of one spacer 406 and one of the spacer grooves 408, where the lens engagement surface 410 is a surface configured to contact an edge of a lens positioned within the system 300. Looking briefly to FIG. 6, FIG. 6 shows the first adjustable locking mechanism and the second locking adjustable mechanism in more detail via an exploded view 600 of the lens receiving section 304.

As may be seen at FIG. 6, the first adjustable locking mechanism comprising the internal engagement section 402 and the locking ring 404 is shown. The internal engagement section 402 may comprise a plurality of grooves 414 and ridges 412 that form a spiral threading. As to the locking ring, the locking ring 404 may comprise an edge 602 that is compatible with the internal engagement section 402 threading so that the locking ring 404 may be screwed up and down in a direction parallel to the central axis. In this way, lenses with different heights may be accommodated via the system 300.

The locking ring 404 may further comprise one or more notches 603 formed into a surface of the locking ring 404 that faces towards the central axis 310. Such notches 603 may be diametrically opposed, for example.

As to the second type of adjustable locking mechanism comprising the spacers 406 and spacer grooves 408, the spacers 406 are configured to slide into the spacer grooves 408. The spacers 406 may each include an extension 608 and a lens engaging section 604, where a length of the extension 608 may be varied. That is, although the length shown for the extension 608 in FIG. 6 may be the length of the extension 608, in other examples a different length may be used for the extension 608. In this way, lenses with different diameters may be accommodated via the system 300. For example, a longer extension 608 may allow lenses with smaller diameters to be held in place within the lens receiving section 304, and a shorter extension 608 for the spacers 406 may allow lenses with larger diameters to be held in place within the lens receiving section 304. Further, as illustrated at FIG. 6, the extension 608 for all of the spacers 406 may be the same or substantially the same length. Alternatively, in one or more examples, the length of the extensions 608 may differ among the spacers 406 coupled within the lens receiving section 304. In this way, further adaptability to various lenses may be enabled.

As shown in FIG. 6, there may be multiple spacers 406 and corresponding spacer grooves 408. Though there are four of the spacers 406 and corresponding spacer grooves 408 shown in the example at FIG. 6, it is noted that more or fewer of the spacers 406 and spacer grooves 408 are possible. In one or more examples, there may only be one spacer groove 408 or no spacer grooves 408. In examples where there are no spacer grooves 408, it is noted that the lens receiving section 304 does not comprise the second type of adjustable locking mechanism.

As illustrated in FIG. 6, the spacer grooves 408 formed into the lens receiving section 304 shown at FIG. 6 may be approximately equidistant from each other along a circumference of the lens receiving section 304. Alternatively, it is also possible for the spacer grooves 408 to not be equidistant from each other, or for there to only be one or no spacer grooves 408. The spacer grooves 408 may each comprise a cross-sectional profile that is configured to receive one of the spacers 406. For example, the spacers 406 and the spacer grooves 408 may be configured so that the spacers 406 may slide into and out of the spacer grooves 408 in a direction parallel to the central axis 310, while preventing the spacers 406 from moving out of the spacer grooves 408 in a direction perpendicular to the spacer grooves 408.

Looking to the spacers 406, the spacers 406 may each comprise a lens engaging section 604, a groove engaging section 606, and an extension 608. The lens engaging section 604 may include the lens engagement surface 410, where the lens engagement surface 410 is configured to directly contact an edge of a lens when the lens is coupled within the system 300. When the spacers 406 are positioned in the spacer grooves 408, the lens engagement surface 410 of each of the spacers 406 may face inward towards the central axis 310 of the lens receiving section 304.

As to the groove engaging section 606, the groove engaging section 606 may comprise a cross-sectional profile configured to fit into one of the spacer grooves 408 formed into the lens receiving section 304. In one or more examples, the cross-sectional profile of the groove engaging section 606 may be the same shape as the cross-sectional profile of the spacer grooves 408. For example, the cross-section of the groove engaging section 606 and the corresponding groove engaging section 606 may comprise substantially circular cross-sectional profile. Other shapes for the cross-sectional profile of the spacer grooves 408 and the groove engaging section 606 of the spacers 406 may include one or more of other rounded shapes, rectangular, triangular, star, and organic shapes, among others. The cross-sectional shape of the groove engaging section 606 of the spacers 406 and the cross-sectional shape of the spacer grooves 408 allow the spacers 406 to move in a direction parallel to the central axis 310 but not perpendicular to the central axis 310.

An additional feature of the lens receiving section 304 is that it comprises the fourth ring section 328 (also referred to herein as a tactile ring, a lip, or protruding ring surface). In an assembled state (such as shown at FIG. 3), the fourth ring section 328 and the second ring section 324 (also referred to herein as a further tactile ring, further lip, or a further protruding surface) are configured such that a user's fingers may fit comfortably there between, aiding the user in balance while holding the system 300 and preventing slippage.

Thus, as seen at FIG. 6, the lens receiving section 304 may comprise an internal engagement section 402 in the form of an internal wall that is grooved in a spiral form, allowing the locking ring 404 to be screwed down to conform to the height of the examination condensing lens being used. In addition, the internal engagement section 402 may comprise multiple spacer grooves 408 (e.g., four grooves) that are configured to couple with spacers 406 to accommodate lenses of various diameters.

Via the lens receiving section 304 and associated adjustable locking mechanisms, a user may select and insert one of the spacers 406 into each of the spacer grooves 408. The user may then insert their own condensing lens into the opening defined by the lens receiving section 304 and center the lens so that spacers 406 secure the lens in place and there is no motion of the lens in the radial direction taking place. The user may then screw in the locking ring 404 until it pushes against the condensing lens, securing the lens in place and preventing motion along the central axis 310 from taking place. Once the lens is secured, the lens receiving section 304 and the light source section 302 may be connected by screwing the lens receiving section 304 and the light source section 302 together.

Turning back now to FIG. 4, as illustrated, the light source section 302 of the system 300 comprises the light source 206, also referred to herein as the light ring 206. For example, the light ring 206 may be coupled within the light source section 302 of the casing 303. The light ring 206 may comprise fiber optic cables 308, a fiber optic support structure (discussed in further detail herein below), and an electronic board unit 416. The electronic board unit 416 may be a printed circuit board (PCB), in one or more examples.

As previously discussed in relation to FIG. 3, the fiber optic cables 308 may circumferentially surround a light receiving aperture 207 at a second end 424 of the fiber optic cables 308. It is noted that the second end 424 of the fiber optic cables 308 is also referred to as an emission end herein. In at least one example, the second end 424 of the fiber optic cables 308 may be rounded, as shown in FIG. 4. In this way, light emitted from the light ring 206 may be directed into the patient's eye, reflected off of the fundus of the patient's eye, and the light reflected off of the fundus of the patient's eye may be directed through the light receiving aperture 207 and to the lens receiving section 304 of the system 300 to be passed through a lens positioned within the lens receiving section 304.

Continuing with discussion of the light ring 206, the fiber optic cables 308 of the light ring 206 may extend from the end face 312 of the system towards the electronic board unit 416. For example, the fiber optic cables 308 may be supported by the fiber optic support structure, where the fiber optic support structure may include a first support ring 418 and a second support ring 420. It is noted that the fiber optic cable support structure may further include one or more support arms 422 coupled to the first support ring 418 and the second support ring 420. The fiber optic cables 308 supported by the first support ring 418 and the second support ring 420 may extend such that a first end 426 (shown at FIG. 5, for example) of the fiber optic cables 308 opposite the second end 424 terminates near the electronic board unit 416. The first end 426 of the fiber optic cables 308 may also be referred to as a light source receiving end herein.

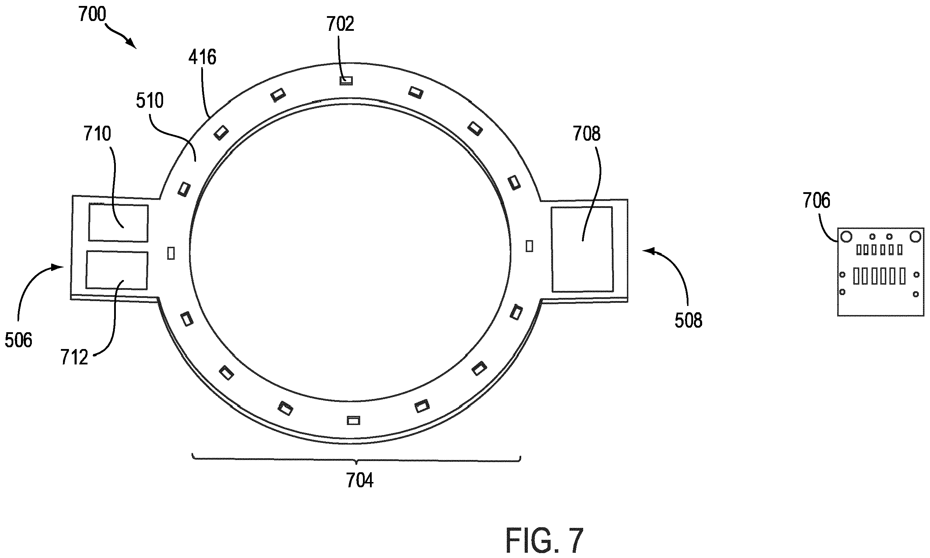

Looking now to FIG. 500, FIG. 5 shows an exploded view 500 of the system 300, and FIGS. 6-12 provide additional views of the components shown in the exploded view 500. As may be seen, FIG. 5 shows the lens receiving section 304, and it is noted that the exploded view at FIG. 6 of the lens receiving section 304 is discussed herein above. As to the remaining features shown in the exploded view 500 at FIG. 5, discussion will move back and forth between the exploded view 500 at FIG. 5 and the additional views provided in FIGS. 7-12.

Thus, looking at FIG. 5, the electronic board unit 416 and the fiber optics support structure 502 are shown. As seen in FIG. 5, the fiber optics support structure 502 may comprise the first support ring 418 and the second support ring 420, where the first support ring 418 comprises a first surface 504 that faces towards the electronic board unit 416. The electronic board unit 416 is generally ring-shaped with a first arm 506 and a second arm 508 extending from the ring. Further, the electronic board unit 416 comprises a light surface 510 that faces towards the first surface 504. Turning briefly to FIG. 7, FIG. 7 shows a view 700 of the light surface 510 of the electronic board unit 416 that faces towards the first surface 504 of the first support ring 418.

As may be seen in FIG. 7, the light surface 510 of the electronic board unit 416 may comprise a plurality of lights 702. The plurality of lights 702 are positioned on a ring-shaped portion 704 of the electronic board unit 416. In at least one example, the plurality of lights 702 may be spaced apart at regular distances around the ring-shaped portion 704. That is, the plurality of lights 702 may be substantially equidistant from one another in one or more examples. In other examples, it is noted that the plurality of lights 702 may not be equidistant from one another, however. The plurality of lights 702 may be light-emitting diodes (LEDs), in one or more examples. In one or more examples, the plurality of lights 702 may comprise a series of LEDs configured to generate a series of colors including one or more of green, yellow, and white with sufficient luminosity carried by the fiber optic cables 308 for emission via the light ring 206 to concentrate the light into the patient's eye.

The electronic board unit 416 may be rotatable within the light source section 302 of the casing 303, in one or more examples. For example, the first arm 506 and the second arm 508 allow the electronic board unit 416 to be manually rotated. It is noted that the first arm 506, the second arm 508, and a USB power board 706 may be positioned within the second ring 324 of the light source section 302 (see FIG. 3, for example).

In at least one example, the first arm 506 and the second arm 508 protrude out of the casing, allowing for the ring to be rotated, and aligning other color LED lights with the fiber optic cables 308. The electronic board unit 416 may be connected via wires to the USB power board 706, and the USB power board 706 may be located in a cavity that extends inside the casing. It is noted that the charging port 319 is a female plug hole within the casing where the electronic board unit 416 may reside, in plane with the opening, as indicated by the charging region 318. The wires connecting the electronic board unit 416 and the USB power board 706 may go through an internal wall of the unit and be out of the way from the optical path of the light. This allows the electronic board unit 416 with the LEDs (plurality of lights 702) to sit above the fiber optics support structure 502, receive power from the USB power board 706 (the USB power board 706 positioned approximately at the middle of the fiber optic support structure 502) without getting in the way.

Rotation of the electronic board unit 416 may allow for contact of the electronic board unit 416 with the USB power board 706, in at least one example. Contact of the electronic board unit 416 with the USB power board 706 may be carried out via one or more of a first contact area 710 and second contact area 712 that are wired for common 708, so that rotation of the electronic board unit 416 may complete a circuit with the USB power board 706. By completing the circuit with the USB power board 706 via either the first contact area 710 or the second contact area 712, the plurality of lights 702 may be energized so that light is emitted via the plurality of lights 702. Additionally, or alternatively to rotation of the electronic board unit 416, a physical switch or microcontroller may be used to activate the different lights 702 and control their operation.

Looking back now to FIG. 5, the light that is emitted via the plurality of lights 702 on the light surface 510 of the electronic board unit 416 is directed towards the fiber optics support structure 502. It is noted that the fiber optic cables 308 are positioned in the fiber optics support structure 502 when the system 300 is in an assembled state. In particular, the second end 424 of the fiber optic cables 308 may be positioned within light emission openings 306 formed into the second support ring 420, and the first end 426 of the fiber optic cables 308 may be positioned within the openings 512 formed into the first support ring 418. In at least one example, the light emission openings 306 are formed into surface 514 of the second support ring 420 and extend through the second support ring 420 to the surface 309 of the second support ring 420. In this way, light may be provided to the first end 426 of the fiber optic cables 308 at the first support ring 418 from the plurality of lights 702, travel down the fiber optic cables 308 to the second end 424 at the second support ring 420, and be emitted from the second end 424 of the fiber optic cables 308.

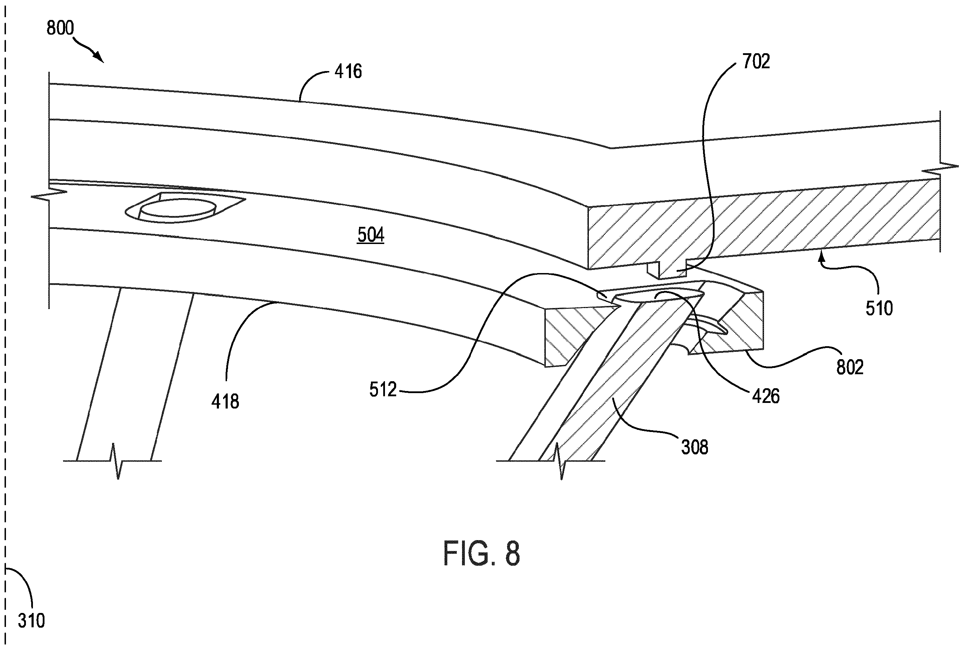

For example, looking briefly to FIG. 8, the plurality of lights 702 may be positioned in direct alignment with the first end 426 of the fiber optic cables 308. That is, the plurality of lights 702 may align with the first end 426 of the fiber optic cables 308 in a direction substantially parallel to the central axis 310. FIG. 8 shows a partial cutaway view 800 of the light source configuration from the binocular eye fundus viewing system 300. It is noted that reference to the light source configuration herein refers to the light source section 302 of the casing 303, as well as all associated components coupled thereto and housed therein to emit the light from the light ring 206.

As may be seen in FIG. 8, the openings 512 formed into the first support ring 418 may go all the way through the first support ring 418. For example, the openings 512 may extend through the first support ring 418 from the first surface 504 of the first support ring 418 to a second surface 802 of the first support ring 418. It is noted that the second surface 802 may be opposite the first surface 504 and face away from the electronic board unit 416. The openings 512 may be sized to hold the fiber optic cables 308 in place, with the first end 426 of each of the fiber optic cables 308 positioned in one of the openings 512. As such, the first end 426 of the fiber optic cables 308 faces a corresponding light of the plurality of lights 702. In one or more examples, it is noted that the first end 426 of the fiber optic cables 308 may be cut at approximately a 47 degree angle, in order to increase an amount of light received at the fiber optic cables 308 via corresponding lights 702.

The openings 512 may be in direct alignment with the plurality of lights 702, in a direction that is parallel to the central axis 310. Further, the first support ring 418 may be positioned less than a predetermined threshold distance away from the plurality of lights 702. In this way, light is efficiently provided from the plurality of lights 702 to the first end 426 of the fiber optic cables 308 positioned within the openings 512.

Continuing now to FIG. 9 and FIG. 10, FIG. 9 shows a perspective view 900 of the fiber optics support structure 502 and FIG. 10 shows a partial cutaway view 1000 of the fiber optics support structure 502.

For purposes of viewing, the fiber optics support structure 502 is shown without the fiber optic cables 308 positioned in the fiber optics support structure 502 at FIG. 9, while the fiber optics structure 502 is shown with the fiber optic cables 308 positioned therein at FIG. 10. FIG. 9 and FIG. 10 are described together.

The fiber optics support structure 502 is configured to support the fiber optic cables 308 so as to funnel light from each of the plurality of lights 702 (e.g., LEDs) via the fiber optic cables 308 for emission at the light ring 206.

In particular, as seen in FIG. 9, the fiber optics support structure 502 may comprise a first support ring 418 and a second support ring 420. The second support ring 420 comprises the surface 309 which (as seen at FIGS. 3 and 4) forms part of an exterior of the system 300 when the system 300 is in an assembled state. As shown in FIG. 9, the surface 309 of the second support ring 420 may be raised relative to a recessed surface 902 of the second support ring 420. That is, a height of the second support ring 420 may be greater at the surface 309 than a height of the second support ring 420 at the recessed surface 902. Additionally, or alternatively, the recessed surface 902 may be concentric with the surface 309. In particular, the recessed surface 902 may be a ring-shaped surface that is concentric with the surface 309 of the second support ring 420. Further, as shown in FIG. 9, the light emission openings 306 that are formed into the surface 309 may extend to the recessed surface 902.

In one or more examples, the surface 309 and the recessed surface 902 of the second support ring 420 may form a mating configuration with the light source section 302 of the casing 303. For example, the recessed surface 902 of the second support ring 420 may be in face sharing contact with an interior surface of the light source section 302 at the first ring section 322 of the casing 303 (see FIG. 4, for example).

Continuing, as further seen at FIG. 9, the first support ring 418 may be larger than the second support ring 420. Due to the difference in size between the first and second support rings 418, 420, when the fiber optic cables 308 are positioned in the fiber optics support structure 502, the fiber optic cables 308 angle inward towards the central axis 310 of the system 300.

The angle of the fiber optic cables 308 inward towards the central axis 310 may be seen at FIG. 10, for example, where FIG. 10 shows a partial cutaway view 1000 of the fiber optics support structure 502 with the fiber optic cables 308 positioned therein.

As seen in FIG. 10, the fiber optic cables 308 may extend from the openings 512 formed around the circumference of the first support ring 418 to the light emission openings 306 formed around the circumference of the smaller, second support ring 420. The configuration of the larger first support ring 418 and the smaller second support ring 420 is based at least in part on optical measurements that allow for the light to be focused by the fiber optic cables 308 into the pupil of the patient's eye, such as pupil 202 with reference to FIG. 2, while also allowing light reflected off the eye fundus of the patient, such as fundus 202 with reference to FIG. 2, to be received via the light receiving aperture 207 and directed through the light source section 302 to the lens receiving section 304 without obstruction.

In one or more examples, the first support ring 418 may have an internal diameter in a range of approximately 40 mm to 50 mm (e.g., 45 mm), and the second support ring 420 may have an internal diameter in a range of approximately 10 mm to 20 mm (e.g., 15 mm). It is noted that the internal diameter of the first support ring 418 refers to a diametric distance measured across the first support ring opening 904 from an internal wall 1002 of the first support ring 418. The internal diameter of the second support ring 420 refers to a diametric distance measured across the light receiving aperture 207 from an internal wall 1004 of the second support ring 420. As shown in FIG. 10, the internal wall 1004 may be a beveled wall, for example. In such cases where the internal wall 1004 is a beveled wall, the internal diameter refers to the portion of the internal wall 1004 that results in the smallest internal diameter. These above-discussed internal diameter ranges for the first support ring 418 and the second support ring 420 may allow for light from the plurality of lights 702 to be focused to a light point of approximately 4 mm to 9 mm, for example 6 mm to 8 mm, which may be the approximate size of a dilated pupil. Further, the internal diameter ranges for the first support ring 418 and the second support ring 420 may allow for light reflected of the eye fundus of the patient to follow a light path through the light receiving aperture 207 and to the lens receiving section 304 of the system 300 without obstruction.

Continuing with FIG. 9 and FIG. 10, as shown, the fiber optics support structure 502 to guide the fiber optic cables 308 in an overall funnel or cone shape. In one or more examples, the fiber optics support structure 502 may follow a predetermined cone semi-angle, where a value of the cone semi-angle impacts a distance away from the patient's eye the system 300 needs to be held for obtaining a clear view of the eye fundus. For example, the cone semi-angle for the fiber optics support structure 502 may be in a range of approximately 92 degrees to 96 degrees. The predetermined cone semi-angle may be based on a lens being used in the lens receiving section 304, in one or more examples. For example, the fiber optics support structure 502 may comprise a cone semi-angle of 94 degrees. By the fiber optics support structure 502 comprising a cone semi-angle of 94 degrees, it was found (when using a traditional ophthalmic condensing lens in the lens receiving section 304) that the system 300 could be positioned exactly 40mm distance away from the eye surface of the patient to achieve particularly high quality eye fundus images. Thus, based on the particular lens to be used or use case (such as physiological variations, for example), the dimensions of the fiber optics support structure 502 may be modified to obtain a cone semi-angle for the desired distance from the eye surface to the system 300 to obtain a high quality view.

Continuing with FIG. 9 and FIG. 10, the second support ring 420 is further configured to enable a tip angle of each of the fiber optic cables 308 to be adjusted within an adjustment range, wherein the tip angle refers to an angle of the second end 424 of the fiber optic cables 308. That is, the second support ring 420 is configured so that the second end 424 of each of the fiber optic cables 308 can move in an adjustment range of approximately 5 mm towards the central axis 310 to 5 mm away from the central axis 310 from a base position. The base position for the fiber optic cables 308 may be a position at which the fiber optic cables 308 extend straight from the first support ring 418 to the second support ring 420, without the second end 424 of the fiber optic cables being deflected.

The tip angle for the second end 424 of the fiber optic cables 308 may be adjusted via individual deflection devices and/or a deflection ring device. An example of a configuration for individual deflection devices is shown at FIG. 10. As may be seen in FIG. 10, the second support ring 420 may comprise deflection device bores 1006, where each of the deflection device bores 1006 opens into one of the light emission openings 306. For example, each of the deflection device bores 1006 may extend in a radial direction that is substantially perpendicular to the central axis 310, from an internal wall 1004. In cases where the internal wall 1004 may be a beveled wall, for example, the deflection device bores 1006 may be formed into a portion of the internal wall 1004 that flares out, away from the central axis 310.

As previously discussed, and as shown in FIG. 10, the second end 424 of the fiber optic cables 308 extend through the light emission openings 306. Thus, the second end 424 for the fiber optic cables 308 are accessible via the corresponding deflection device bores 1006. For example, individual deflection devices may be inserted into one or more of the deflection device bores 1006 to contact the second end 424 of the fiber optic cables 308. In this way, the tip angle at the second end 424 for each of the fiber optic cables 308 may be individually adjusted. The more the individual deflection device pushes on the second end 424 of one of the fiber optic cables 308, the more the tip angle may be changed.

In one or more examples, one or more of the deflection device bores 1006 may comprise a threaded engagement section configured to receive an individual deflection device. The individual deflection device may be a screw (e.g., a flat bottom screw), a pin, or other suitable device, for example. In cases where the individual deflection device may be a screw, one or more screws may be screwed into one or more of the deflection device bores 1006 to deflect the second end 424 of corresponding fiber optic cables 308. In this way, the tip angle of the individual fiber optic cables 308 at the second end 424 may be adjusted by the user, thus allowing light emitted at the second end 424 of the fiber optic cables 308 as part of the light ring 206 to be focused and concentrated as desired.

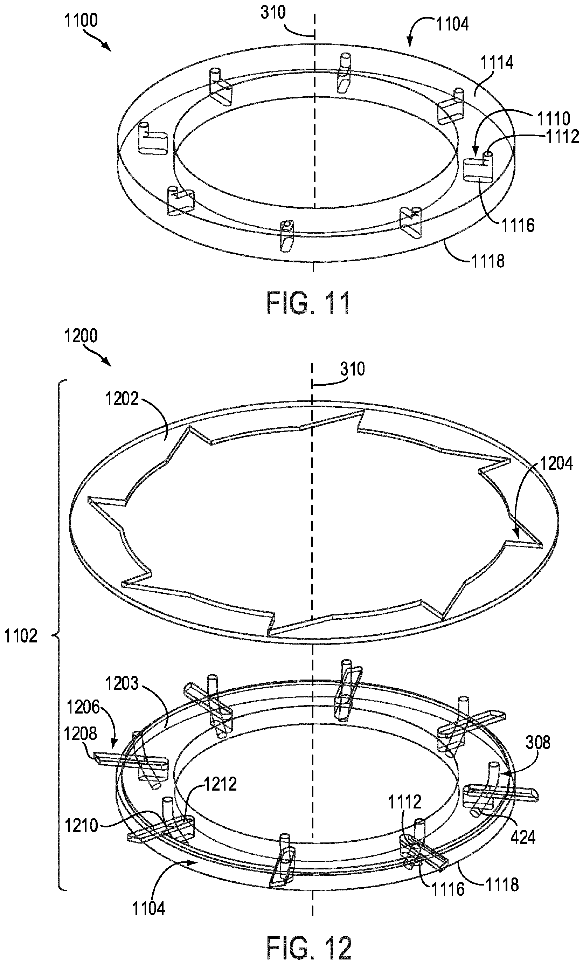

Additionally, or alternatively to the individual deflection devices, a deflection ring device as shown in FIGS. 11-13B may be included to adjust the tip angle at the second end 424 of the fiber optic cables 308. For example, looking to FIG. 11, FIG. 11 shows a view 1100 of a base ring 1104.

As may be seen at FIG. 11, the base ring 1104 may comprise fiber optic cavities 1110. The fiber optic cavities 1110 may be cavities that are formed within base ring 1104 and provide an opening that goes through the base ring 1104. For example, fiber optic cavities 1110 may comprise a first opening 1112 formed into a first surface 1114 of the base ring 1104 and a second opening 1116 formed into a second surface 1118 of the base ring 1104. As shown in FIG. 11, the first opening 1112 may be a substantially circular cross-section, and the second opening 1116 may be a rounded and elongated opening.

In one or more examples, the first opening 1112 may be approximately the size of the fiber optic cables 308 and may thus limit movement of a portion of the fiber optic cables 308 positioned at the first opening 1112. In contrast, the second opening 1116 may be sized to allow for movement of a portion of the fiber optic cables 308 that are positioned at the second opening 1116.

In at least one example, the first surface 1114 may face away from light ring 206 end of the system 300, and the second surface 1118 may face towards the light ring 206 end of the system. In other words, the first surface 1114 may face away from the direction of light emission, while the second surface 1118 may face towards the direction of light emission in the system 300. The fiber optic cables 308 may be positioned within the fiber optic cavities 1110 such that the second end 424 is positioning in the second opening 1116. Thus, in this way, the fiber optic cavities 1110 formed into the base ring 1104 may be configured to allow movement of fiber optic cables 308 at the second end 424.

For purposes of being able to view the fiber optic cavities 1110, it is noted that the base ring 1104 at FIG. 11 has been shown separately from other components of the deflection ring device. As may be seen at FIGS. 12-13B, the base ring 1104 is a component of the deflection ring device 1102 that may be implemented with additional components to enable the angle of the fiber optic cables 308 to be adjusted at the second end 424. For example, looking to FIG. 12, an exploded view 1200 of the deflection ring device 1102 is shown.

As seen in FIG. 12, the deflection ring device 1102 comprises a rotatable ring 1202, a deflection ring 1203, and the base ring 1104. When assembled, the rotatable ring 1202 may circumferentially surround the deflection ring 1203 and the base ring 1104. The rotatable ring 1202 may comprise notches 1204 that engage deflectors 1206 of the deflection ring 1203. As shown in FIG 12, the notches 1204 may be triangular in shape, in at least one example. However, other shapes are possible for the notches 1204, so long as the notches 1204 are able to effectively engage with the deflectors 1206 of the deflection ring 1203.

The deflectors 1206 of the deflection ring 1203 may be extensions that have a notch engaging end 1208 and a cable engaging end 1210. The cable engaging end 1210 of the extension partially defines deflection openings 1212 that are formed into the deflection ring 1203. The deflection openings 1212 at least partially overlap with each of the first openings 1112 of the fiber optic cavities 1110 in an axial direction.

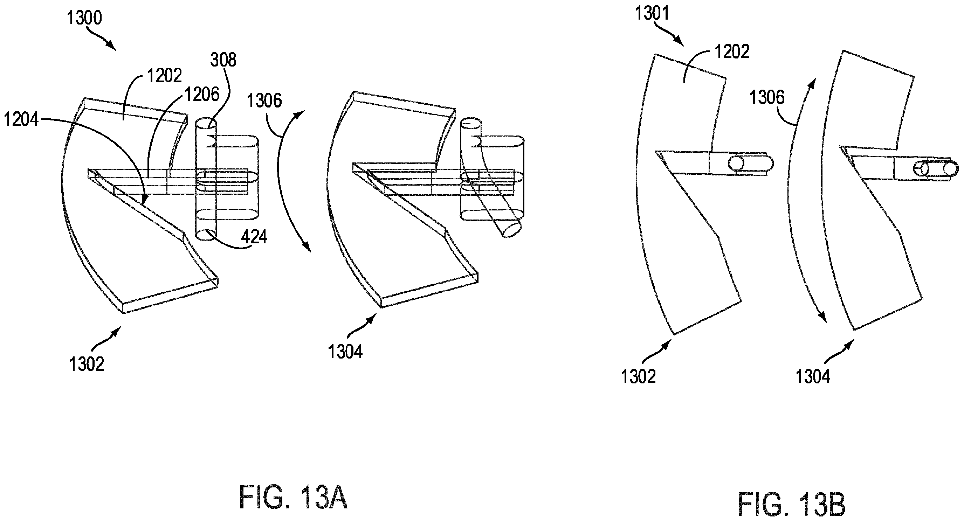

In one or more examples, the deflection ring 1203 may be rotatable relative to the base ring 1104. In this way, the fiber optic cables 308 may be deflected and the tip angle at the second end 424 of the fiber optic cables 308 may be adjusted to change a concentration of the light emitted via the system 300. For example, looking to FIG. 13A and FIG. 13B, FIG. 13A shows a first partial view 1300 of an angle adjustment carried out via the deflection ring device 1102. As to FIG. 13B, FIG. 13B shows a second partial view 1301 of the angle adjustment carried out via the deflection ring device 1102. In particular, the first partial view 1300 may be a partial perspective view, and the second partial view 1301 may be a partial top view.

As may be seen in FIG. 13A and FIG. 13B, the rotatable ring 1202 rotated as indicated via double headed arrow 1306 from a first position 1302 to a second position 1304. The rotatable ring 1202 may be rotated about the central axis 310, in one or more examples.

As the rotatable ring 1202 is rotated from the first position 1302 to the second position 1304, the rotatable ring 1202 engages the deflector 1206 and deflects a portion of one of the fiber optic cables 308. For example, as may be seen in FIG. 13A the deflection of one of the fiber optic cables 308 results in the second end 424 of the fiber optic cable 308 moving from a first, substantially straight position at 1302 to an angled position at 1304. In this way, rotation of the rotatable ring 1202 adjusts the tip angle at the second end 424 of one of the fiber optic cables 308.

Via one or more of the individual deflection devices and the deflection ring device, focusing of light can be done as many times as needed. For example, the light may be focused based on the light intensity or environmental conditions that may affect luminosity and focus. The ends of the fiber optic may further be rounded in order to further focus the light (see FIG. 3, for example).

In this way, via the individual deflection devices and/or the deflection ring 1102, the light emitted from the light ring 206 via the fiber optic cables 308 may be concentrated as desired by a user. By being able concentrate the light emitted from the light ring 206, variations for a particular patient examination may be accommodated. For example, one or more of physiological variations, lens variations (e.g., variations in condenser lens focusing), diffraction, refraction, and other optical properties and variables may be accommodated.

Due to the ability to adjust the tip angle of each of the fiber optic cables 308, the technical effect of enabling the light emitted to be fine-tuned as needed to generate improved image quality is achieved. Additionally, the technical effect of a compact and adaptable system for providing a binocular view of an eye fundus of a patient is achieved. In this way, the accessibility of BIO examinations may be improved.

This focusing of light may be done as many times as desired, based on the light intensity or environmental conditions that may affect luminosity and focus, for example. The combination of different LED colors, luminosity, and ability to adjust a focus of the light ring allows the light to be manipulated to create a better image, and adapt to environmental conditions and the optical needs of different condensing lenses, cameras, or other image capturing devices (if such image capturing devices are used).

Turning back now to FIG. 5, for reference, it is noted that the electronic board unit 416, fiber optics support structure 502, and fiber optic cables 308 may be contained within the light source section 302 of the system 300. Further, the individual deflection devices and/or the deflection ring device 1102 may be positioned within the light source section 302. For example, the deflection device bores 1006 illustrated at FIG. 10 may be formed into the second support ring 420 within the light source section 302. Additionally, or alternatively, the deflection ring device 1102 illustrated at FIG. 12 may be positioned in the light source section 302.

In at least one example, the deflection ring device 1102 illustrated at FIG. 12 may be an alternative to the second support ring 420. In other examples, the deflection ring device 1102 may be incorporated immediately upstream or immediately downstream of the second support ring 420. In one or more examples that include the deflection ring device 1102, it is noted that an opening may be formed into the light source section 302 to all for rotation of the rotatable ring 1202 when the system 300 is in an assembled state.

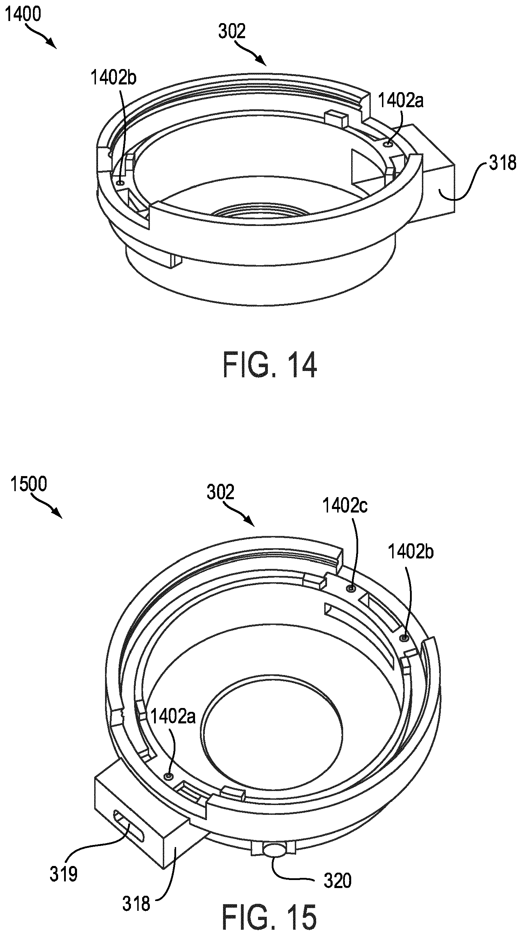

Looking at the light source section 302 in more detail, attention is directed to FIG. 14 and FIG. 15. FIG. 14 shows a first view 1400 of the light source section 302, and FIG. 15 shows a second view 1500 of the light source section 302.

As may be seen in FIG. 14 and FIG. 15, the light source section 302 may comprise one or more spring loaded contacts, including a first spring loaded contact 1402a, a second spring loaded contact 1402b, and a third spring loaded contact 1402c. The spring loaded contacts, such as the first spring loaded contact 1402a, second spring loaded contact 1402b, and third spring loaded contact 1402c, may be configured to contact the first contact area 710 and the second contact area 712 on the electronic board unit 416 (see FIG. 7). The first spring loaded contact 1402a, second spring loaded contact 1402b, and third spring loaded contact 1402c may be energized.

As the ring-shaped electronic board unit 416 is rotated, and one or more of the first contact area 710 and the second contact area 712 make contact with the spring loaded contacts housed inside the light source section 302 of the casing 303, the plurality of lights 702 are controlled. This is not least because wires for the plurality of lights 702 are channeled from within the light source section 302 contact the USB power board 706, where the connector for the USB power board 706 is housed within the light source section 302. In this way, based on the position of the electronic board unit 416 relative to the spring loaded contacts, the plurality of lights 702 on the electronic board unit 416 may be controlled.

Looking to FIG. 16 and FIG. 17, for example, FIG. 16 shows a first example circuit diagram 1600 and FIG. 17 shows a second example circuit diagram 1700 that may be implemented to control the plurality of lights 702, in one or more examples. The first example circuit diagram 1600 helps to illustrate an example color mode selection. For example, the plurality of lights 702 on the electronic board unit 416 may comprise two colors of LEDs arranged in LED pairs 1602. For example, a first LED pair 1602a may comprise a first color LED 1604a (e.g., yellow) and a second color LED 1606a (e.g., green). Similarly, a second LED pair 1602b may comprise the first color LED 1604b (e.g., yellow) and the second color LED 1606b (e.g., green). As shown in the first example circuit diagram 1600 at FIG. 16, along with a current limit resistor 1608 in series, the LED pairs 1602 may be in parallel with other pairs of the same type of LED, energizing and controlling their behavior simultaneously.

The positive end of the LEDs of both colors may be connected together, from common 708. The common 708 may be +5V. The negative end of each color LED circuit may connect to its respective contact area (e.g., the first contact area 710 and the second contact area 712). Thus, by rotating the electronic board unit 416, the contact area completing the circuit may be changed and in turn change the color emitted.

For polarity reversal operation, rather than rotation of ring-shaped electronic board unit 416, it is noted that the system may have green, white, or yellow LEDs in a back to back configuration (pairs) in one package, sharing the same current limit resistor. In such examples, +5VDC may be applied by operating a switch, such as user input device 320. In such examples, an input to the switch turns on one color. Reversing the polarity turns on the other color via another input to the switch. Lastly, removing power turns the LEDs off. Further, in one or more examples, the LEDs may be green and yellow (or white- yellow, white-green, etc. in pairs) elements in a dual color LED package, but electrically isolated so they can be connected in series pairs.

The second example circuit diagram 1700 at FIG. 17 helps to illustrate an example intensity control selection. That is, the second example circuit diagram 1700 shows at least one example for adjusting a brightness of the light emitted.

Overall, the primary purpose of the circuit disclosed herein is to provide the necessary power to the LEDs (plurality of lights 702). Thus, without departing from the scope of the present disclosure, it is understood that the power supply could provide for either +5 vdc (such as USB power) or +12 vdc (for example, a battery pack). Switching between the two LED circuits (shown as green and yellow) may be accomplished by rotating the LED circuit board. Spring loaded pins (which may be referred to as pogo pins) making contact with tinned areas of the circuit board perform the switching function.

Each pair of LEDs (e.g., yellow shown or green) may share a series resistor whose purpose is to limit the current to below the LED manufacturers specifications.

A MOSFET is incorporated to provide PWM (Pulse Width Modulation) for the purpose of controlling the LED current (intensity). This is done via the "PWM IN" through a MOSFET gate resistor as shown in the diagram.

To provide an LED current feedback for the electronics, a low value MOSFET source resistor is provided. An op-amp amplifier circuit amplifies the voltage drop developed across the source resistor to provide a usable "Current Out" analog voltage (e.g., between 0 and +5 vdc) as an input to a microcontroller.

Looking now to FIG. 18, FIG. 18 shows a flow chart of an example method 1800, according to one or more examples of the present disclosure. The method 1800 may be carried out via the system 300, in one or more examples. Thus, example components of the system 300 that may be used to implement the method 1800 are also described herein.

At step 1802, method 1800 comprises directing light from a light source at a patient-facing end of the system directly to an eye of a patient. For example, the light may be directed on a light path from the light ring 206 to the eye of the patient without the light being passed through a lens (e.g., a condenser lens). Further, it is noted that the light directed to the eye of the patient along the light path may be adjusted and focused via one or more of the approaches disclosed herein. For example, one or more of a color, intensity, and focus of the light may be adjusted.

After step 1802, step 1804 may comprise receiving light reflected from a fundus of the eye via a light receiving aperture. That is, the light path of the light directed from the light source at step 1802 of method 1800 may be reflected off of the fundus of the eye, and then the light may continue along the light path and be directed through the light receiving aperture. The light receiving aperture may be light receiving aperture 207, for example. In this way, the light receiving aperture may be configured to receive the light that has been reflected off the light fundus along the light path.

The light path for the light received via the light receiving aperture at step 1806 may then be directed through a lens receiving section for a single pass through a lens positioned therein. For example, the light reflected off of the eye fundus may be directed through the light receiving aperture, through an unobstructed path that extends along the central axis 310 of the light source section 302 of the casing 303, and into the lens receiving section 304 of the casing 303. As part of the light path through the lens receiving section 304, the light may pass through a lens (e.g., a condenser lens) positioned in the lens receiving section 304 of the casing 303. The light path may then extend out of the lens receiving opening (e.g., lens receiving opening 314) at the practitioner-end of the system 300.

Following step 1806, method 1800 comprises generating a binocular image (e.g., binocular image 218) of the eye fundus at step 1808. The binocular image may be viewed by a user at a practitioner-end of the system 300.

In one or more examples, method 1800 may further comprise a step 1810, which may include capturing the binocular image via an image capture device (e.g., image capture device 222). In particular, the image capture device may be mobile phone, camera, or other image capture device that is positioned in the light path after the light is passed through the lens to capture the binocular image generated at step 1806. In one or more examples, the image capture device may be a separate device from the system 300.

Thus, provided herein are systems and methods to generate a binocular image of an eye fundus via a light ring of LEDs (e.g., light ring 206) and a physical ring (e.g., fiber optics support structure 502) that guides the fiber optic cables (e.g., fiber optic cables 308) through a trough that allows light emanating from this ring to enter the pupil directly and be reflected by the tissues and structures of the fundus, passing only once through the condensing lens, through the opening at the base of the unit (e.g., light receiving aperture 207) in order to generate a binocular image at the incidence angle of view, which is 180 degrees. Unlike prior art systems such as those shown at FIG. 1, the present disclosure binocular image is generated by light passing only once through the lens, without a headset or head/spectacle mounted system. This allows for the practitioner to view the fundus with binocularity and at the same time, enables the practitioner to use a camera, cellular phone, or any other image capturing device to record the image that is viewed. Further, the system is fully portable and may be approximately the size of a tennis ball (or smaller), and may be powered by batteries (including rechargeable USB battery packs) due to the low powered light sources being used. Further, because the light focusing elements are manually controlled in the present disclosure, examination may be performed in a wide variety of environments for patients with varying needs without the use of complicated calibration tools.

The systems and methods disclosed herein may generate a binocular image of the fundus of the eye using a single path of light through the emission of light from a selectable source (e.g., light ring 206) that generates light that is guided by fiber optics (e.g., fiber optic cables 308) housed within a support and guided structure (e.g., fiber optics support structure 502) and focused through mechanical modification of the fiber optic angle so that light is emitted from the base of the system (e.g., at end face 312). In this way, light generated from the light source travels to a patient's eye, passing through the pupil and reflecting from the retina at a fundus of the eye to create a binocular image, all via a hand-held ophthalmic examination lens. This single light pass is in contrast to prior art BIO system headsets, where light travels from an external source, through an ophthalmic examination lens to be reflected by the retina to travel again past the same ophthalmic examination lens (dual pass) for an image to be generated with retaining binocularity (e.g., see FIG. 1).

The system disclosed herein may be composed of a casing (e.g., casing 303) whose top half inner cavity (e.g., lens receiving section 304) is designed to accept common ophthalmic examination condensing lenses with different diameters, thicknesses and power. The lower half portion of the outer casing (e.g., light source section 302) houses the electronic boards, connectors, wires and control switches, while also serving the purpose of being a support and enclosure for the fiber optic housing and aperture control structure. This fiber optic housing is specifically configured to allow fiber optic cables (e.g., fiber optic cables 308) to safely be housed, where the fiber optic cables may be cut at an angle of approximately 47 degrees in order to be parallel to the LED or light source (e.g., the plurality of lights 702) at a top end (e.g., first end 426) in order to have the greatest surface area exposed to the emitted light, and rounded at the bottom (e.g., second end 424 of the fiber optic cables 308) in the base of the unit (e.g., at the end face 312) to concentrate light further. This light exits the system from the base of the unit (e.g., via the light ring 206 at the end face 312) bypassing an ophthalmic examination condensing lens, and allowing for the single path light to be used to view the fundus with binocularity.

The disclosure also provides support for a system for binocular viewing of an eye fundus, comprising: a casing comprising a first section coupled to a second section, a light source coupled to a patient-facing end of the first section and configured to provide light along a light path towards the eye fundus, and a lens receiving opening formed into a practitioner-facing end of the second section of the casing, the lens receiving opening configured to receive and hold a lens within the second section. In a first example of the system, the light path of the light includes reflection of the light off of the eye fundus and back towards the patient-facing end of the first section, and wherein patient-facing end comprises a light receiving aperture configured to receive the light directed on the light path back towards the patient-facing end. In a second example of the system, optionally including the first example, the first section of the casing is detachably coupled to the second section of the casing. In a third example of the system, optionally including one or both of the first and second examples, the system is a binocular indirect ophthalmoscope (BIO) system. In a fourth example of the system, optionally including one or more or each of the first through third examples, the second section comprises an adjustable locking mechanism configured to position and hold the lens within the second section. In a fifth example of the system, optionally including one or more or each of the first through fourth examples, a light receiving aperture opens into the lens receiving opening, wherein the light source is a light ring that surrounds the light receiving aperture, and wherein the light path extends through the light receiving aperture and out of the lens receiving opening. In a sixth example of the system, optionally including one or more or each of the first through fifth examples, the system further comprises: an optical support structure that is positioned within the first section of the casing, wherein the optical support structure guides a plurality of fiber optic cables in a cone shape.

The disclosure also provides support for a binocular indirect ophthalmoscopy (BIO) system, comprising: a casing, the casing comprising a light source section and a lens receiving section, a light ring coupled to the light source section of the casing at a first end, the light ring surrounding a light receiving aperture that is formed into the first end of the light source section, a lens opening defined by the lens receiving section of the casing, the lens opening configured to receive and hold a lens in place. In a first example of the system, the light ring comprises a plurality of fiber optic cables that are guided via an optical support structure, the optical support structure comprising a first support ring and a second support ring. In a second example of the system, optionally including the first example, the light ring comprises a plurality of fiber optic cables, and wherein an angle of the plurality of fiber optic cables is adjustable at the light ring. In a third example of the system, optionally including one or both of the first and second examples, the system further comprises: an adjustable locking mechanism configured to position the lens within the lens receiving section of the casing. In a fourth example of the system, optionally including one or more or each of the first through third examples, the light ring is configured to provide light in a direction away from the lens opening.

The disclosure also provides support for a method for binocular viewing of an eye fundus, comprising: providing light directly from a light source to the eye fundus, receiving the light from the eye fundus at a lens, and generating a binocular image of the eye fundus via the light received at the lens. In a first example of the method, the light is not passed through the lens before being provided to the eye fundus. In a second example of the method, optionally including the first example, the binocular image is generated via a single pass of the light through the lens. In a third example of the method, optionally including one or both of the first and second examples, binocular image is an aerial image, and wherein the aerial image is a true image that is horizontally and laterally inverted. In a fourth example of the method, optionally including one or more or each of the first through third examples, the light source is a light ring. In a fifth example of the method, optionally including one or more or each of the first through fourth examples, the lens comprises a light receiving side and an image viewing side that is opposite of the light receiving side. In a sixth example of the method, optionally including one or more or each of the first through fifth examples, the method further comprises: rotating a ring of the light source to adjust a focus of the light provided via the light source. In a seventh example of the method, optionally including one or more or each of the first through sixth examples, the binocular image is an aerial image.FIGS. 3-15 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example. Additionally, reference to upstream and downstream as disclosed herein references a direction of a path of light. Further, openings shown formed into the components may be described as such.

It will be appreciated that the configurations disclosed herein are exemplary in nature, and that these specific embodiments are not to be considered in a limiting sense, because numerous variations are possible. Unless explicitly stated to the contrary, the terms "first," "second," "third," and the like are not intended to denote any order, position, quantity, or importance, but rather are used merely as labels to distinguish one element from another. The subject matter of the present disclosure includes all novel and non-obvious combinations and sub-combinations of the various systems and configurations, and other features, functions, and/or properties disclosed herein.

As used herein, the term "approximately" is construed to mean plus or minus five percent of the range unless otherwise specified.

The following claims particularly point out certain combinations and sub-combinations regarded as novel and non-obvious. These claims may refer to "an" element or "a first" element or the equivalent thereof. Such claims should be understood to include incorporation of one or more such elements, neither requiring nor excluding two or more such elements. Other combinations and sub-combinations of the disclosed features, functions, elements, and/or properties may be claimed through amendment of the present claims or through presentation of new claims in this or a related application. Such claims, whether broader, narrower, equal, or different in scope to the original claims, also are regarded as included within the subject matter of the present disclosure.

## Claims

1. A system for binocular viewing of an eye fundus, comprising:
a casing comprising a first section coupled to a second section;
a light source coupled to a patient-facing end of the first section and configured to provide light along a light path towards the eye fundus; and
a lens receiving opening formed into a practitioner-facing end of the second section of the casing, the lens receiving opening configured to receive and hold a lens within the second section.

2. The system of claim 1, wherein the light path of the light includes reflection of the light off of the eye fundus and back towards the patient-facing end of the first section, and wherein patient-facing end comprises a light receiving aperture configured to receive the light directed on the light path back towards the patient-facing end.

3. The system of claim 1, wherein the first section of the casing is detachably coupled to the second section of the casing.

4. The system of claim 1, wherein the system is a binocular indirect ophthalmoscope (BIO) system.

5. The system of claim 1, wherein the second section comprises an adjustable locking mechanism configured to position and hold the lens within the second section.

6. The system of claim 1, wherein a light receiving aperture opens into the lens receiving opening, wherein the light source is a light ring that surrounds the light receiving aperture, and wherein the light path extends through the light receiving aperture and out of the lens receiving opening.

7. The system of claim 1, further comprising an optical support structure that is positioned within the first section of the casing, wherein the optical support structure guides a plurality of fiber optic cables in a cone shape.

8. A binocular indirect ophthalmoscopy (BIO) system, comprising:
a casing, the casing comprising a light source section and a lens receiving section;
a light ring coupled to the light source section of the casing at a first end, the light ring surrounding a light receiving aperture that is formed into the first end of the light source section;
a lens opening defined by the lens receiving section of the casing, the lens opening configured to receive and hold a lens in place.

9. The BIO system of claim 8, wherein the light ring comprises a plurality of fiber optic cables that are guided via an optical support structure, the optical support structure comprising a first support ring and a second support ring;
and/or
wherein the light ring comprises a plurality of fiber optic cables, wherein an angle of the plurality of fiber optic cables is adjustable at the light ring.

10. The BIO system of claim 8, further comprising an adjustable locking mechanism configured to position the lens within the lens receiving section of the casing.

11. The BIO system of claim 8, wherein the light ring is configured to provide light in a direction away from the lens opening.

12. A method for binocular viewing of an eye fundus, comprising:
providing light directly from a light source to the eye fundus;
receiving the light from the eye fundus at a lens; and
generating a binocular image of the eye fundus via the light received at the lens.

13. The method as claimed in claim 12, wherein the light is not passed through the lens before being provided to the eye fundus.

14. The method as claimed in claim 12, wherein the binocular image is generated via a single pass of the light through the lens.

15. The method as claimed in claim 12, wherein binocular image is an aerial image, and wherein the aerial image is a true image that is horizontally and laterally inverted.

16. The method as claimed in claim 12, wherein the lens comprises a light receiving side and an image viewing side that is opposite of the light receiving side.
